# EUROPEAN PATENT APPLICATION

(11) **EP 0 877 538 A2**
(43) Date of publication of application: **11.11.1998**
(21) Application number: 98108216.7
(22) Date of filing: 06.05.1998
(51) Int. Cl.: H05G 1/02

(54) **An X-ray apparatus and a method for rearranging such an X-ray apparatus**

(30) Priority: 09.05.1997 DK 53797
(71) Applicant: NRT-NORDISK ROENTGEN TEKNIK A/S, DK-8361 Hasselager (DK)
(72) Inventor: Jensen, Aksel Hjorth, 8220 Brabrand (DK)
(74) Representative: Nielsen, Leif

(57) **Abstract**

There is described an X-ray apparatus that may be rearranged from a right sided to a left sided configuration with the purpose of increasing flexibility and easy access to a patient table (9). The X-ray apparatus has a C-bracket (15) on which an X-ray tube (17) and an image receiver (19) are mounted. The X-ray apparatus has an L-shaped angular bracket (4) with a main arm (5) that is suspended on a base unit (1) and which supports a lateral arm (6) on which the table (9) is mounted.

The table (9) may be extended to a position outside the C-bracket (15), thereby allowing rotation of the C-bracket about a horizontal axis (22). The table (9) is arranged for rotation about a horizontal axis (10) from a first horizontal position in parallel with the main arm (5) to a second horizontal position in parallel with the main arm, but with an opposite orientation of the table (9).

## Description

The present invention relates to an X-ray apparatus comprising a base unit from which an L-shaped angular bracket is suspended, the angular bracket comprising a main arm carrying through a suspension a C-shaped bracket with an X-ray tube at one end and an image receiver at the other end, where a lateral arm mounted on the C-shaped bracket carries a patient table for supporting a person, and where the C-shaped bracket and the angular bracket are arranged for mutual movement in the form of rotation and/or translation.

The invention also relates to a method for rearranging such an X-ray apparatus.

X-ray apparatuses have an X-ray tube at one end of a C-shaped bracket and at the other end of the C-bracket, there is an image receiver. The image receiver may be an image amplifier, a film, or a flat detector with CCD. Between these two components, there is extended a ray path utilised at the examination of a person placed on the patient table. The C-shaped bracket is hereinafter called the C-bracket.

In the X-ray apparatus, the L-shaped angular bracket is fastened to the base unit by rotatably suspending the longer leg of the bracket, the main arm, about a horizontal axis on the base unit by a central position on the main arm. At the same time, the main arm is intended to support a C-bracket carriage comprising a bracket on which the C-shaped bracket is mounted. Hereby the C-bracket may be slided along the main arm and at the same time be pivoted about a horizontal axis in parallel with the rotational axis of the main arm, as the C-bracket is pivotably suspended in the carriage/bracket. At one end of the main arm, the shorter leg of the L-shaped angular bracket, the lateral arm is fastened at its one end. The lateral arm is mounted on the main arm via a bracket making possible a displacement of the patient table in direction against the main arm and away from the main arm. At the free end of the lateral arm, there is mounted a bracket for the patient table. The table extends away from the bracket and mainly in parallel with the main arm. Via the fastening bracket the table may be rotated relative to the lateral arm. This rotation may be between two extreme positions on each side of a horizontal orientation of the table. In normal use, the table will be placed so that a person on the table is situated in the ray path between the X-ray tube and the image receiver.

The ray path may extend in all directions. The X-ray tube may be freely arranged over or under the table with the image receiver situated at the opposite end, under and over the table, respectively.

With the purpose of making examinations under an oblique angle relative to the person on the table, the C-bracket may be pivoted relative to the table (and the main arm). Thus, it is common that the C-bracket may be pivoted about a substantially horizontal axis to both sides relative to a horizontal orientation of the C-bracket. The C-bracket may also be displaced in the longitudinal direction relative to the table, so that the ray path may be placed at different positions along the table. In practice, displacement takes place by sliding the C-bracket carriage on which the C-bracket is mounted along the main arm, which forms a part of the angular bracket on which the patient table is mounted.

It is also possible to rotate the C-bracket in its own plane around the table. Hereby the ray path may move from a vertical to a horizontal orientation and thereby pass through the patient or the person on the table at different angles.

In the know X-ray apparatuses it will also be possible to rotate the table around its longitudinal axis. This takes place by rotating the table in a suspension at the free end of the lateral arm.

X-ray apparatuses will normally be built up so that they are right sided. Alternatively, they may also be built up as left sided. That means that the table is mounted on the angular bracket in a position to the right, or to the left relative to the base unit, respectively, when the X-ray apparatus is seen from the front. That is, when you are looking at the base unit from a position where the C-bracket and the angular bracket are between the base unit and the viewer.

Another example of an X-ray apparatus of the kind mentioned in the introduction is also known. This X-ray apparatus is built up with the table mounted on a lateral arm which is slidable along the main arm together with the C-bracket. This construction will increase the accessibility to the table to a certain degree, because it may be slided to one or the other side in parallel with the main arm. Hereby it becomes possible for an operator to stand on an arbitrary side of the table when an examination is performed. However, this construction will not make possible a rearrangement of the X-ray apparatus so that it is either right sided or left sided. The C-bracket will always be on the same side of the lateral arm. This reduces the accessibility. In this construction, it will not be possible for the user to choose, whether the X-ray tube is to be over or under the patient table.

Once an X-ray apparatus is arranged as a right sided or a left sided X-ray apparatus, it will not be possible to rearrange it. However, there is a wish for an increased flexibility so that the user may choose himself, whether the X-ray apparatus should be right or left sided. Also, it is desired that the user may choose if the X-ray tube is to be over or under the table.

Also, there is a strong wish for better access to the table from both sides, irrespective of whether the table is right or left sided. Thus, it is important that there is access to the table for both a doctor and a nurse. In this situation, the doctor will normally be at one side of the table, while the nurse is at the other side of the table.

It is the purpose with the present invention to indicate an X-ray apparatus having an increased flexibility and easy access to the table, while it has the capability of being rearranged, in a simple way, from a right sided to a left sided X-ray apparatus, and which at the same time has the possibility of an arbitrary orientation of the C-bracket with the X-ray tube above or below the patient table. It is also the purpose to indicate a method for establishing rearrangement of the X-ray apparatus.

According to the invention, this is achieved with an X-ray apparatus of the kind mentioned in the introduction and which is peculiar in that the lateral arm and the table are arranged to be displaced so that the table may be slided away from the ray path in a direction in parallel with the rotational axis of the main arm to a position outside the C-bracket, allowing a rotation of the C-bracket about a horizontal axis in parallel with the axis of rotation of the main arm, that the table is arranged for rotation from a first and substantially horizontal position in parallel with the main arm, with the head end of the table directed in a first direction, to a second and substantially horizontal position in parallel with the main arm and with the head end directed opposite to the first direction.

In practice, the rotation of the table from the first to the second horizontal position will normally take place via a rotation of the main arm and thereby a rotation of the table which is kept affixed in the rotation with the main arm.

With an X-ray apparatus having these peculiar features, it will be possible to perform a rearrangement from a right sided to a left sided X-ray apparatus, or vice versa, with a suitable displacement/pivoting of the elements of the X-ray apparatus in a correct sequence. It will also be possible to change the position of the X-ray tube and the image receiver in relation to the table by a suitable displacement/pivoting of the elements of the apparatus in a correct sequence.

As the apparatus may arbitrarily be right or left sided, it will be possible to achieve an increased accessibility to the table. Helpers and users may thus place themselves either at the head or the foot of the table and at both sides of the table. The X-ray apparatus thus display an increased flexibility and an increased accessibility for the user.

The method according to the invention is peculiar in that the table is put in a starting position where the ray path extends along a substantially straight line through the table, and where the table is mounted on the lateral arm in a horizontal position in parallel with the main arm and with the head end directed in a first direction, that the table is displaced to a position outside the C-bracket, that the table is rotated to a second horizontal position in parallel with the main arm, in which position the head end of the table is directed opposite to the first direction, and that the table is displaced into the ray path.

By this method the X-ray apparatus may be rearranged between a right sided and a left sided position so that the user may arbitrarily choose the configuration of the X-ray apparatus.

It will be possible to perform the rearrangement with the patient lying on the table or to perform a rearrangement before a person is placed on the table.

According a first aspect of the invention, the method is peculiar in that the displacement of the table from the first to the second horizontal position takes place by rotating the main arm and the table mounted thereon through an angle of 180° about an axis which is parallel with the axis of rotation of the C-bracket, that the table is rotated 180° about its longitudinal axis, and that the table is displaced into the ray path.

By this method it will be possible to perform a rearrangement between a right sided and a left sided apparatus of the kind where the table is mounted on a lateral arm connected to the main arm with a suspension at one end. The rearrangement will preferably take place without any person on the table. The rearrangement only requires very little space around the X-ray apparatus. The requirement for space is further reduced, if the patient table is rotated to a vertical position in its position outside the C-bracket.

According to a second aspect of the invention, the method is peculiar in that the table is displaced in direction in parallel with the main arm until it is outside the ray path, that the table is rotated 180° about a vertical axis, and that the table is slided into the ray path.

With this method it will be possible to rearrange an X-ray apparatus from being right sided to being left sided, or reversely, when the X-ray apparatus is of the kind where the patient table is mounted on a lateral arm which is sliding along the main arm, either with the C-bracket or independently of the C-bracket. With this method, it will be possible to perform a rearranging with the person placed on the table as this is only rotated in a horizontal plane.

Further advantages and special features by the apparatus will appear from the description below with reference to the attached drawing, where
- Fig. 1: shows a simplified view of an embodiment of an X-ray apparatus according to the invention as seen from the front,
- Fig. 2: shows the X-ray apparatus in Fig. 1 from the side,
- Fig. 3: shows the X-ray apparatus in Fig. 1 from above,
- Fig. 4: shows a simplified perspective view of an X-ray apparatus according to the invention,
- Figs. 5-7: show simplified perspective views of the X-ray apparatus shown in Fig. 4 with elements placed in different positions,
- Figs. 8-11: show simplified views corresponding to Fig. 1 of the X-ray apparatus as seen from the front with the elements of the apparatus placed in four different operating modes,
- Figs. 12-28: show schematic drawings of two side views for illustrating the rearranging of the apparatus with a method according to the invention,
- Fig. 29: shows a simplified perspective view of second embodiment of an X-ray apparatus according to the invention, and
- Fig. 30: shows a view from the side of the X-ray apparatus shown in Fig. 29.

In the following, identical or corresponding elements forming a part of the X-ray apparatus will be denoted with the same reference. Thus, there will not be given any specific explanation to each element in connection with each of the Figures of the drawing.

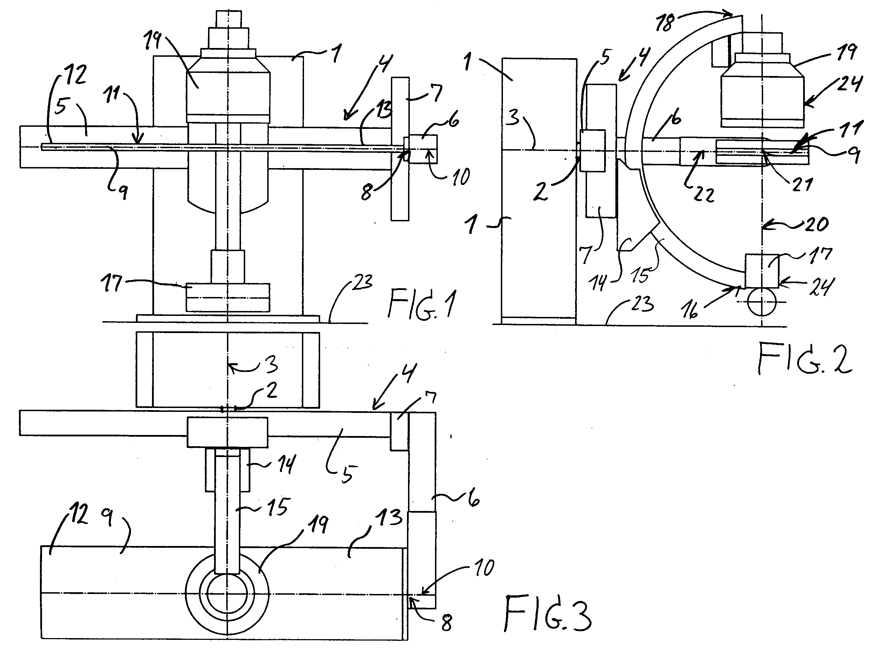

Reference is made firstly to the Figures 1-3. The X-ray apparatus has a base unit in the shape of a casing, where there is provided an axle 2 having an axis of rotation 3. The axle 2 projects from the front of the base unit 1 and may in a way known by itself be slided in the base unit in a vertical direction. An L-shaped angular bracket 4 is mounted on the axle 2 and is thus arranged to be rotated about the axis 3. The angular bracket 4 is formed by a main arm 5 and a lateral arm 6. The lateral arm 6 is telescopic and formed by two tubular profiles which may be slided mutually. The lateral arm 6 is mounted on the main arm 5 via a bracket 7 which makes it possible to slide the lateral arm 6 in a vertical plane relative to the main arm 5.

The lateral arm 6 supports a patient table 9 via a suspension 8 so that the table may rotate about an axis 10 extending perpendicularly on the axis of rotation 3 of the main arm. In the shown configuration, the X-ray apparatus is right sided as the lateral arm 6 is placed at the right side of the apparatus as seen from the front. The table 9 has a top side 11 which may be plane or slightly curved and where a patient normally may be supported with the head placed to the left at the head 12 and the legs at the foot 13 of the patient table 9.

On the main arm 5, there is suspended a C-shaped bracket, abbreviated C-bracket 15 via a bracket 14. The C-bracket has at its one end 16 an X-ray tube 17 and at its other end 18 an image receiver 19 which in the shown embodiment is constituted by an image amplifier. As alternative to the image amplifier, there may also be used a film or a flat detector with CCD. Between the X-ray tube 17 and the image receiver 19, there is a ray path 20 which is to pass through the person placed on the table 9. The bracket 14 may be slided together with the C-bracket 15 along the main arm 5 so that the ray path 20 may be disposed arbitrarily on the table 9 between the head 12 and the foot 13. The bracket 14 and the C-bracket 15 are provided with interacting rail means making it possible to rotate the C-bracket 15 in its own plane between extreme positions defined by the dimensions of the arch of the C-bracket. In the shown embodiment, the dimension of the arch of the C-bracket 15 is about 180°. Rotation of the C-bracket 15 takes place about a centre 21 formed by the C-bracket 15. The C-bracket 15 may rotate about a horizontal axis 22 that is parallel with the axis of rotation 3 of the main arm 5. In the shown position of main arm and C-bracket, the rotational axes 3 and 22 are coincident.

The base unit 1 is placed on a floor surface 23. The floor surface 23 will, together with walls and ceiling in the room in which the X-ray apparatus is placed, define the limits of the room that is available for using and rearranging the X-ray apparatus between its different positions. It is desired that the use of the X-ray apparatus requires as little room as possible in order to reduce the need for space as much as possible.

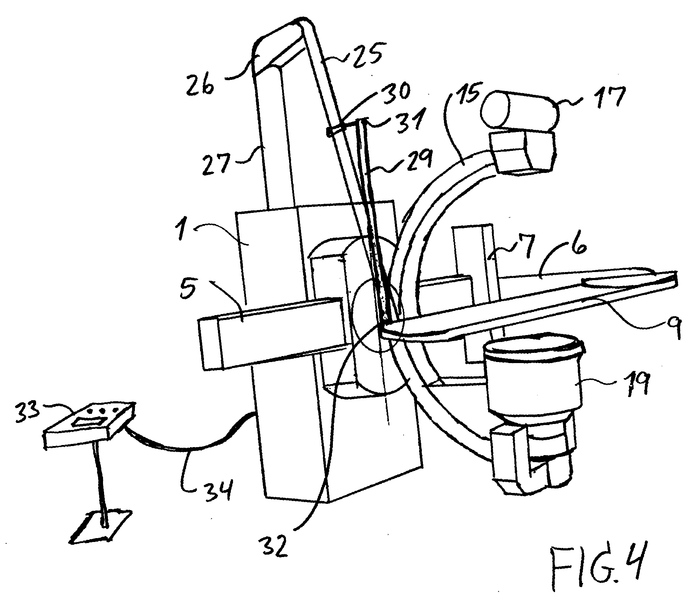

The lateral arm 6 is arranged to perform a displacement so that the patient table 9 may be slided outside the outermost part 24 of the X-ray tube/image receiver 17,19. This allows a rotation of the C-bracket about the axis of rotation 22. The C-bracket is arranged for rotating at least 180° about the axis of rotation 22. In practice, the C-bracket 15 may rotate more than 360° about the axis 22. The rotation of the X-ray tube about the axis of rotation 22 will be limited by the cable connections 25 (see Figure 4) to the X-ray tube 17 and the image receiver 19. In Figures 1-3 the cable connections 25 are not shown for the sake of clarity. As alternative to the cable connections 25, there may be used connections via slide shoes in the suspension of the C-bracket 15 instead. If slide shoes are used, the C-bracket may rotate freely. However, it will be unsuitable for practical reasons to use slide shoes because of the large stresses.

As it appears from Figs. 4-7, the cable connections 25 extend from the top side 26 of a gallows 27 which is situated at the top of the base unit 1. The cable connection is connected with the C-bracket via a connecting box 28. The cable connections 25 are supported by a cable arm 29. The cable arm 29 is mounted via a bracket 30 largely at the middle of the cable connection 25. The cable arm is provided with a hinge connection 31 between the cable arm 29 itself and the connecting bracket 30. The cable arm 29 has furthermore a hinge link 32 between the cable arm and the bracket 14 on which the cable arm is mounted. The cable arm 29 is arranged for always being directed upwards so that it keeps the cable connection 29 in a position above the main arm 5 when this is rotated about its rotational axis 3.

The X-ray apparatus furthermore comprises a control unit which is built into the base unit. The control unit is connected via cables 34 to a control panel 33 that for the sake of clarity is illustrated only in Figure 4. The control unit is arranged to control the desired movements. The control unit is furthermore provided with pre-programmed stop codes for the movement of the different elements. These stop codes are dependent on the physical dimensions of the elements of the apparatus and on the room in which the apparatus is placed.

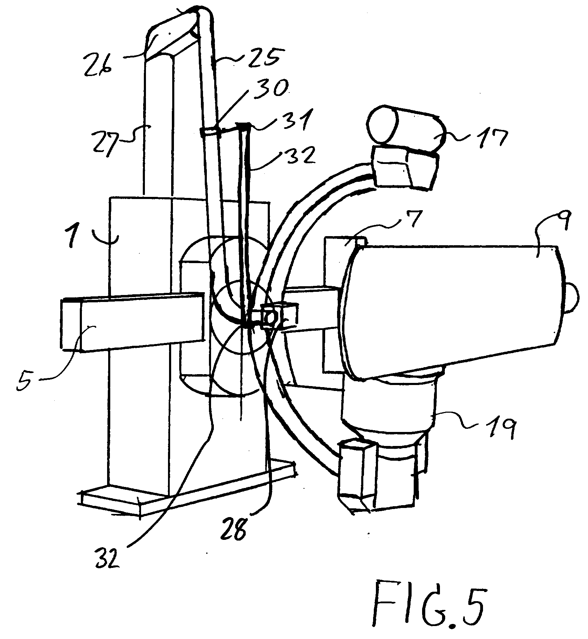

In Figs. 4-7 the X-ray apparatus is seen in a right sided position. In Figures 4 and 5 the X-ray tube 17 is placed above the patient table 9, while the image receiver 19 is placed below. In Figure 4 the table 9 is placed in a horizontal orientation in the ray path between the X-ray tube and the image receiver. In Fig. 5 the lateral arm 6 is displaced to a position outside the C-bracket and at the same time the table 9 is rotated through 90° about the axis of rotation 10 so that it is situated with a vertical orientation. It is noted that the table 9 may be displaced further out with a greater extension of the lateral arm 6. This will make possible a subsequent rotation of the C-bracket. However, there will be a lesser need for space, when the table 9 is rotated to a vertical position as shown in Figure 5 during the subsequent rotation of the C-bracket 15 about its axis of rotation 22.

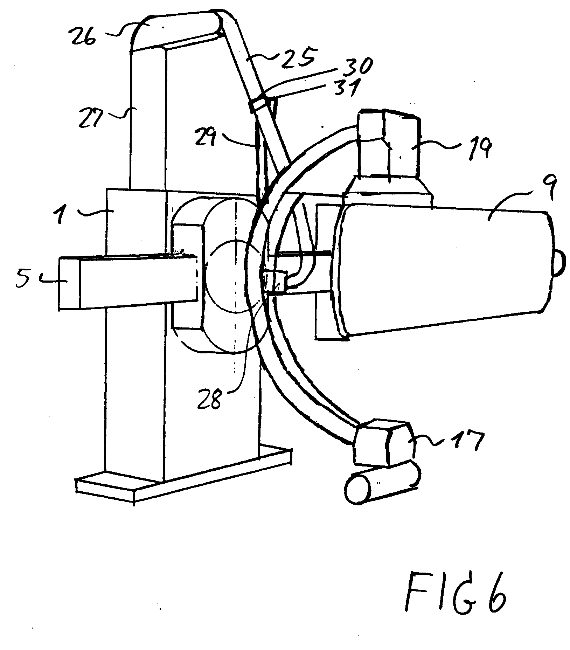

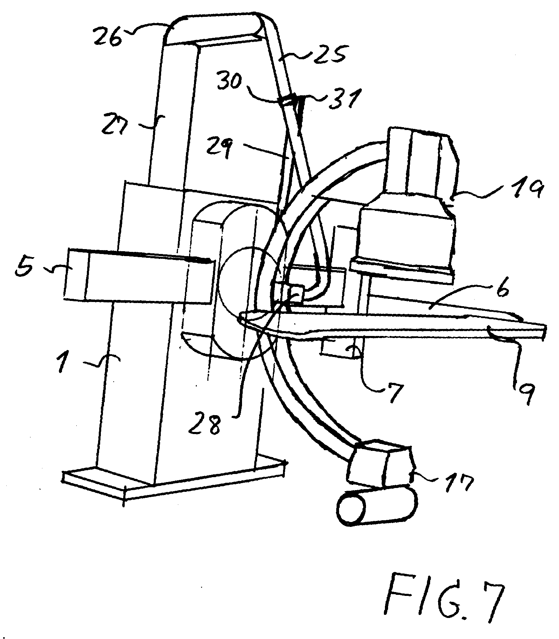

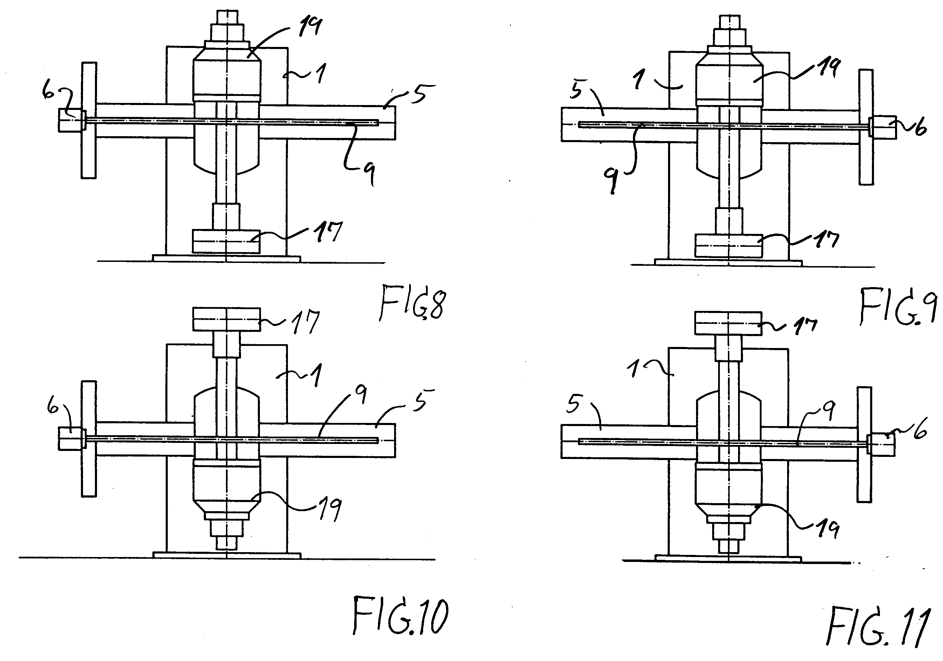

In Figure 6 the X-ray apparatus is seen after rotating the C-bracket 15 180° about the axis 22. Hereby the X-ray tube 17 is disposed below the table 9, while the image amplifier 19 is disposed above the table 9. In Figure 7 the table 9 is rotated 90° about the axis of rotation 10 and the lateral arm 6 is pulled telescopically together so that the table 9 again is situated in the ray path 20 between the X-ray tube 17 and the image receiver 19.

From Figures 4-7 it also appears that the cable arm 29 at the rotation is rotated from its position in Figures 4 and 5 to the left of the C-bracket as seen from the front to a position illustrated in Figures 6 and 7 to the right of the C-bracket.

In Figures 8-11 there is shown four different mutual positions of the elements of the X-ray apparatus. These Figures thus illustrate flexibility and the possibility of using the X-ray apparatus in different ways.

Figures 8 and 9 show the X-ray apparatus with the X-ray tube 17 provided below the table 9 and image receiver 19 provided above the table. Figure 8 shows a left sided X-ray apparatus, as the lateral arm 6 on which the table 9 is suspended is to the left when the X-ray apparatus is viewed from the front. In Figure 9, the X-ray apparatus is seen a right sided configuration with the lateral arm 6 placed to the right, when the X-ray apparatus is viewed from the front.

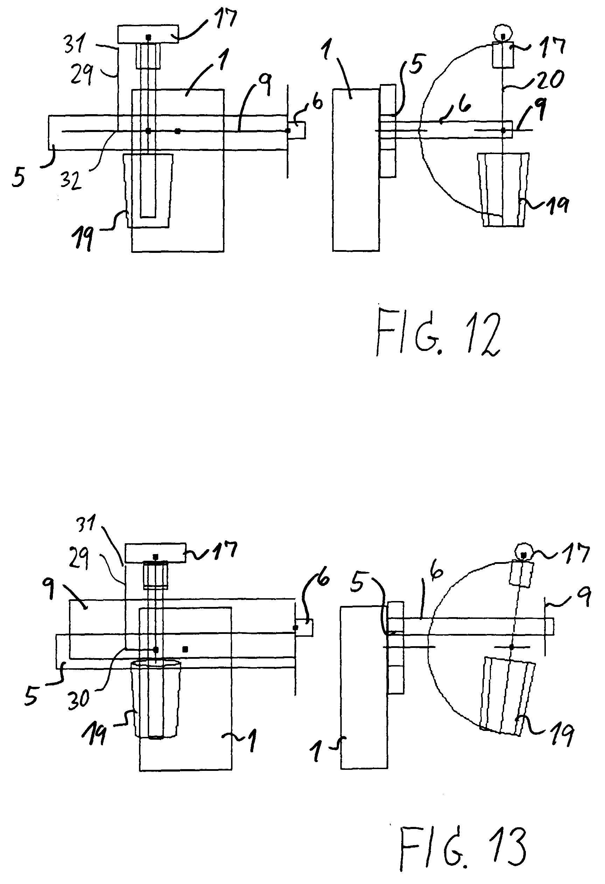

In Figures 10 and 11 the X-ray apparatus is seen in a similar way in a left sided and a right sided configuration. In Figures 10 and 11 the X-ray apparatus is illustrated with the X-ray tube 17 provided above the table 9 and the image receiver 19 below the table.

In the following there will be given an explanation of a method for rearranging an X-ray apparatus between the different modes of operation shown in Figures 8-11. The following explanation will be given with reference to Figures 12-28.

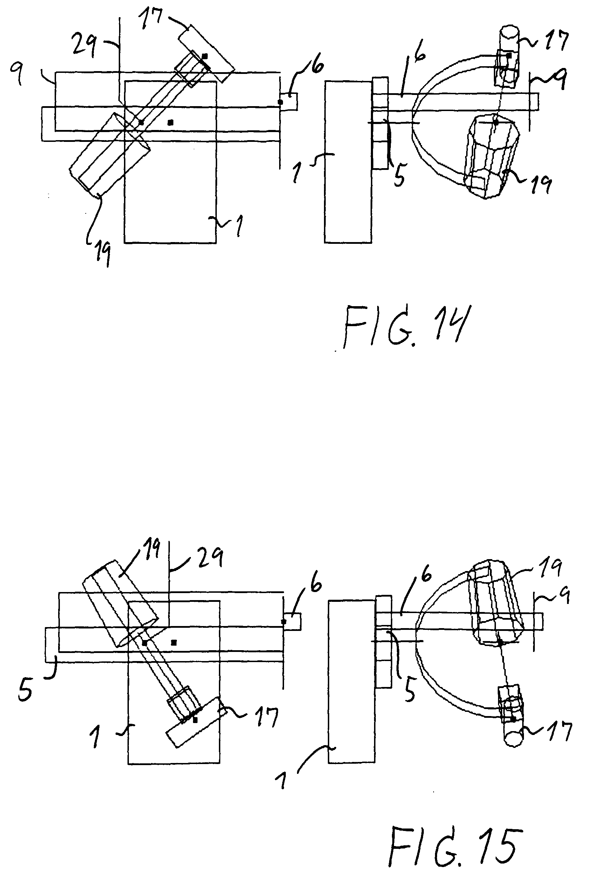

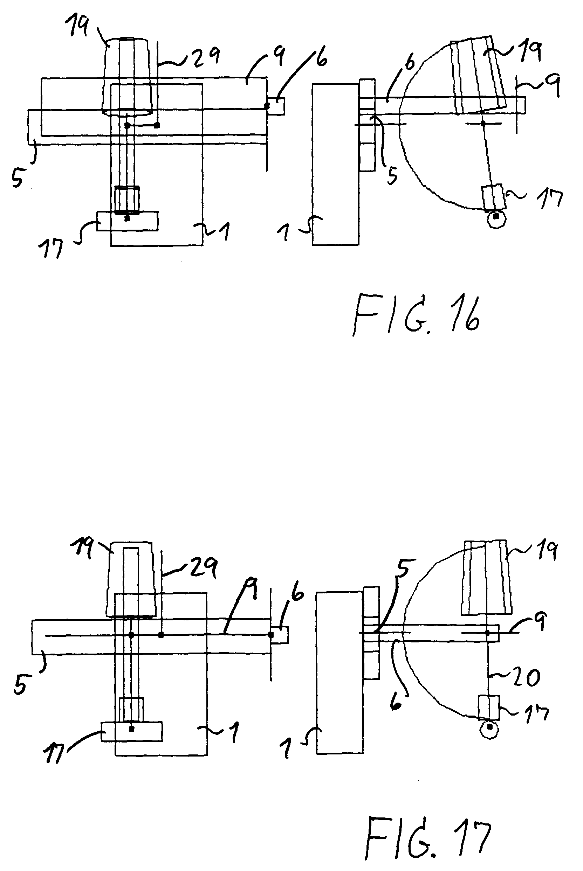

Firstly, the rearranging of the X-ray apparatus is described in which the X-ray tube and the image receiver interchange their positions. In Figure 12 is seen a starting position with the X-ray apparatus in a right sided position with a vertical ray path and the X-ray tube 17 disposed above the table 9 (corresponding to Figure 4). The table 9 is extended to a position outside the C-bracket 15 and is rotated to a vertical position shown in Figure 13. Hereafter the C-bracket 15 is rotated (clockwise) about the axis of rotation 22. The C-bracket may be pivoted within the angular bracket 4, as the lateral arm is situated in a radius from the axis of rotation 22 greater than the largest radius for the C-bracket 15. The X-ray tube 17 may thus be swung from the position shown in Figure 14 to the position shown in Figure 15, and further on to the position shown in Figure 16, where the ray path is vertical again, but where the positions for the X-ray tube and the image receiver are interchanged. Hereafter the table 9 is rotated and slided into the ray path 20 to the position shown in Figure 17. The X-ray apparatus has thus been through a rearrangement from the position shown in Figure 11 to the position shown in Figure 9.

Hereafter, there is described a way in which the X-ray apparatus is rearranged from being right sided to being left sided.

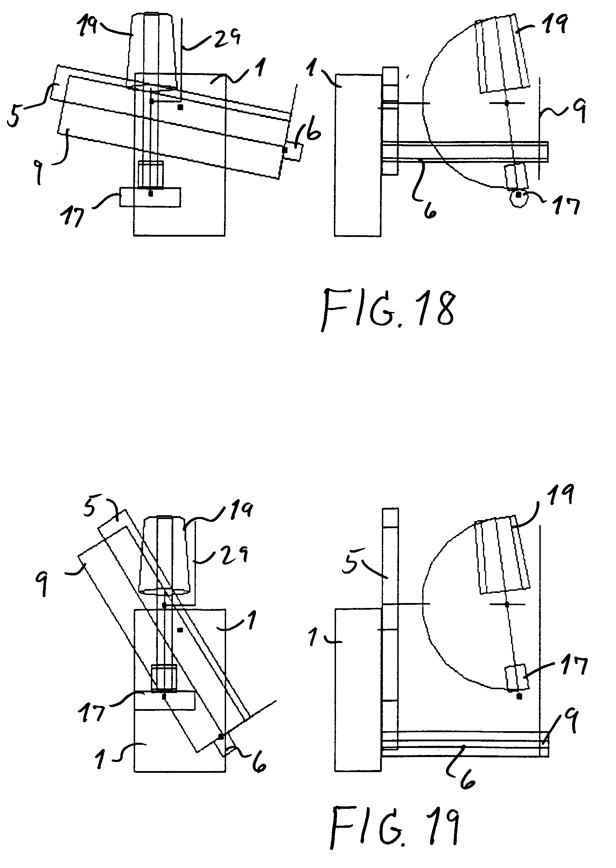

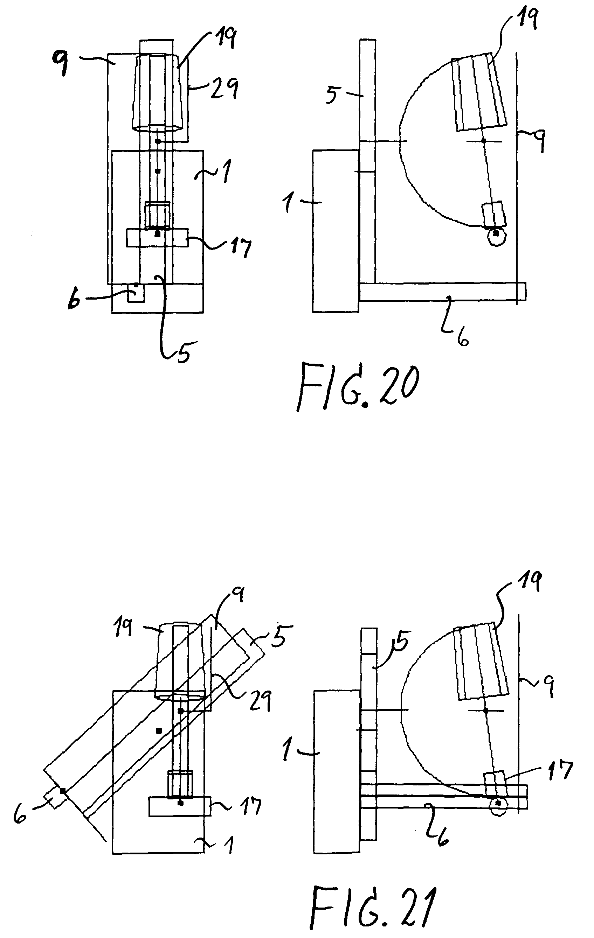

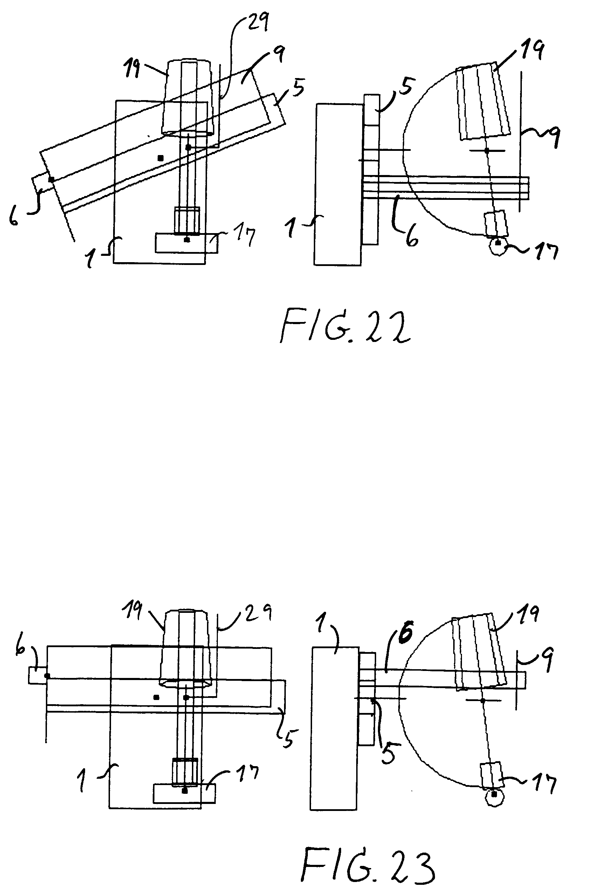

From a position shown in Figure 17, the table 9 is displaced outside the C-bracket and rotated to the vertical position. Hereafter, the angular bracket 4 and the patient table 9 mounted thereon are rotated about the axis of rotation 3. During the rotation, the C-bracket is kept in a constant vertical orientation. It is to be noted that before rotating the angular bracket 4, there is performed a displacement in the vertical direction of the angular bracket in relation to the base unit 1 so that the outermost part of the lateral arm 6 will go free of floor, walls, and ceiling in the room, where the X-ray apparatus is placed. The angular bracket 4 is pivoted through the positions shown in Figures 18, 19 and 20. It is to be noted that the cable arm 29 is kept in a vertical position. It is also to be noted that the cable arm 29 has such a length that the connecting bracket 30 is in a radius so that the free end of the main arm passes under the cable connections 25 from the position shown in Figure 20 to the position shown in Figure 21 during the rotation of the angular bracket 4 about the rotational axis 3. The angular bracket 4 is rotated further on through the position shown in Figure 22 to the position shown in Figure 23. Then the patient table 9 is rotated to a horizontal position and the lateral arm 6 is pulled telescopically together so that the patient table 9 is again situated in the ray path 20. The patient table is now situated in a left sided configuration (corresponding to Figure 8).

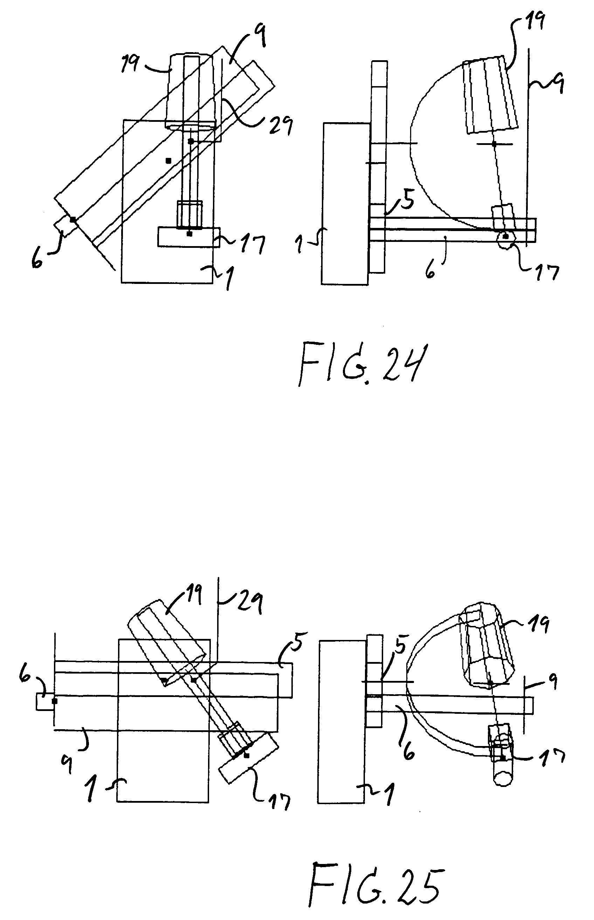

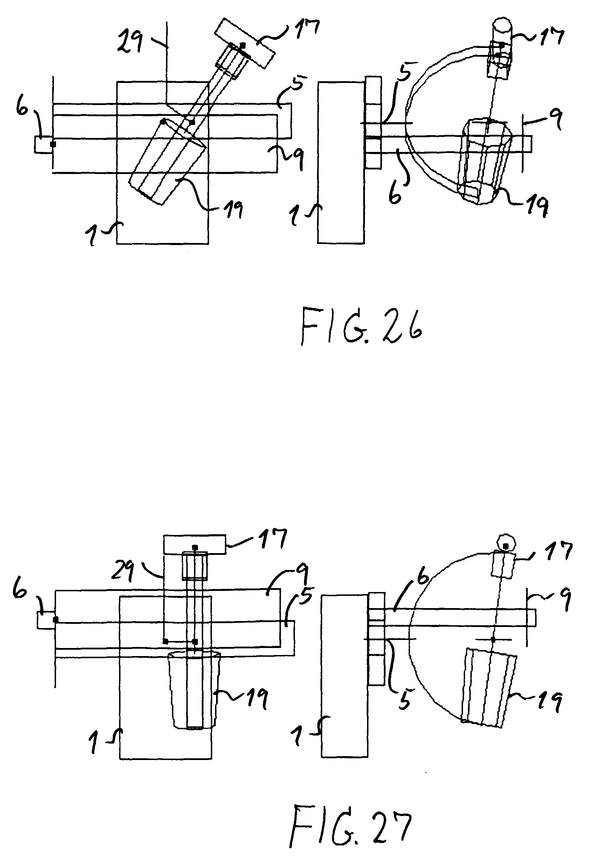

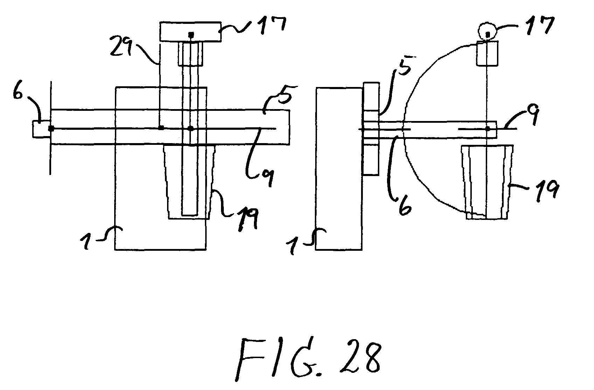

From the position shown in Figure 24, the patient table may again be extended outside the C-bracket and rotated to a vertical position, whereafter the C-bracket may be rotated 180° through the position shown in Figures 25 and 26 to the position shown in Figure 27, where the C-bracket 15 is placed in a vertical plane again, but with the X-ray tube 17 uppermost. Hereafter the table is rotated to a horizontal position and displaced into the ray path 20 to the position shown in Figure 28 (corresponding to Figure 10).

As it appears from the above, the X-ray apparatus according to the invention may be rearranged from left sided to right sided and vice versa. In both of these positions, it will also be possible to rearrange the X-ray apparatus so that the X-ray tube 17 is over or under the table 9 with the image receiver 19 placed at the opposite side of the patient table 9.

Besides the shown configurations it will also be possible to rotate the C-bracket in a way known by itself about a horizontal axis so that the C-bracket 15 is rotated in its own plane. Hereby the ray path 20 may be put into different angular orientations from vertical to horizontal depending on the dimensions of the arch of the C-bracket 15. If this rotation of the C-bracket 15 takes place by a position at the end of the table, it will be possible subsequently to rotate the C-bracket 15 about the axis of rotation 22. Hereby the C-bracket will swing from a position below the patient table to a position over the table. Hereafter the C-bracket 15 may again be rotated back in its own plane about the horizontal axis going through the centre 21 of the C-bracket. In this way, it will also be possible to change the position of the X-ray tube/image receiver above and below the table 9, respectively.

All of the movements described above may be performed automatically as a consequence of signals from the control unit or manually by activating handles (not shown) on the control panel 33.

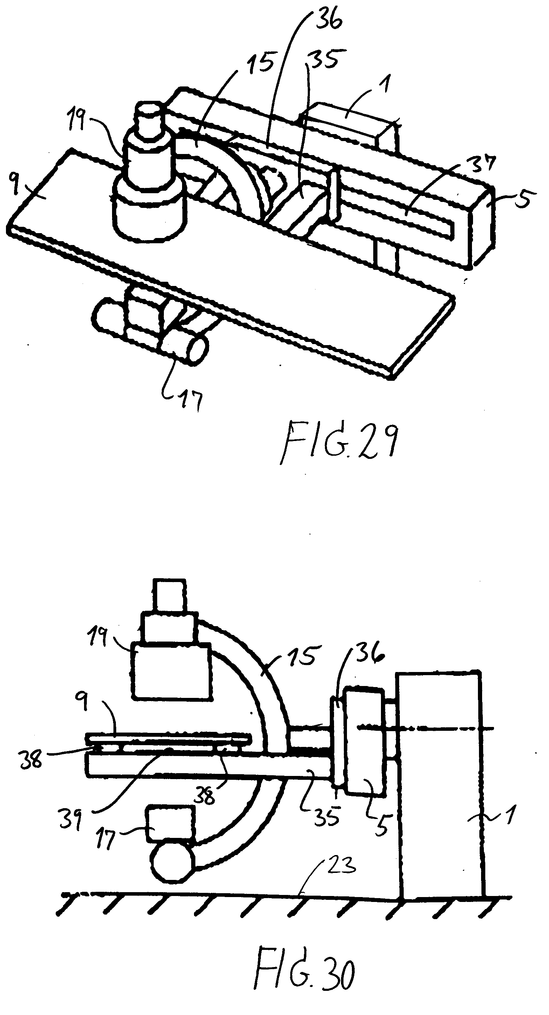

In Figures 29 and 30, there is illustrated a second embodiment of an X-ray apparatus according to the invention. This X-ray apparatus differs from the above embodiments by having the patient table suspended on a lateral arm 35 mounted on a bracket 36 which at the same time supports the C-bracket 15 and which is arranged for sliding along the main arm 5 via a rail track 37. The patient table 9 is mounted via rails 38 on the lateral arm 35 so that the table 9 may be slided in relation to the lateral arm 35 in a direction in parallel with the longitudinal axis of the main arm 5. The rails 38 are mounted in a rotatable suspension 39 in relation to the lateral arm 35. Hereby the table 9 may be rotated in relation to the lateral arm 35 through 180°. In this way the positions of the head 12 and the foot 13 of the table may be interchanged.

The rotation of the table 9 takes place by sliding this to an outermost position, where the lateral arm 35 is situated under the foot or the head end. Hereafter the rotation is performed. Then the table may again be brought to its position within the C-bracket so that the ray path 20 passes through a person on the table 9. With this embodiment of the X-ray apparatus, there will be a need for more space in front of the X-ray apparatus in order to make possible the rotation of the table between a right sided and a left sided position. However, it will be possible to perform a rearrangement between right sided and left sided, if this is desired, while a person is on the table 9.

The shown embodiments of the X-ray apparatus may freely be right sided or left sided, and there will be easy access to the table 9 from its front side as well as its back side irrespective of whether the X-ray apparatus is right sided or left sided. Thus, it will be easy for the operating personnel to perform examination of a person on the table 9 irrespective of whether the examination is to be performed at the legs or the head of the patient.

## Claims

1. An X-ray apparatus comprising a base unit from which an L-shaped angular bracket is suspended, the angular bracket comprising a main arm carrying through a suspension a C-shaped bracket with an X-ray tube at one end and an image receiver at the other end, where a lateral arm mounted on the C-shaped bracket carries a patient table for supporting a person, and where the C-shaped bracket and the angular bracket are arranged for mutual movement in the form of rotation and/or translation, **characterised** in that the lateral arm and the table are arranged to be displaced so that the table may be slided away from the ray path in a direction in parallel with the rotational axis of the main arm to a position outside the C-shaped bracket, allowing a rotation of the C-shaped bracket about a horizontal axis in parallel with the axis of rotation of the main arm, that the table is arranged for rotation from a first and substantially horizontal position in parallel with the main arm, with the head end of the table directed in a first direction to a second and substantially horizontal position in parallel with the main arm and with the head end directed opposite to the first direction.

2. An X-ray apparatus according to claim 1 and of the kind where the lateral arm is suspended from the main arm at one end, wherein the table, with the purpose of rotation between the first and the second positions, is arranged for rotation from the first position about an axis perpendicular to the main arm and coincident with the axis of rotation of the C-shaped bracket through an angle of 180°, as the lateral arm is situated at a radius greater than the greatest radius of the C-shaped bracket, and where the table in this situation is arranged to rotate at least 180° about its longitudinal axis.

3. An X-ray apparatus according to claim 1 and of the kind where the table is mounted on a lateral arm sliding along the main arm either together with the C-shaped bracket or independent of the C-shaped bracket, wherein the table with the purpose of the rotation between the first and second positions is supported on the lateral arm via a suspension allowing the table to be slided relative to the lateral arm in direction in parallel with the longitudinal axis of the main arm, and which is arranged for rotation of the table relative to the lateral arm through 180° about a vertical axis

4. An X-ray apparatus according to claim 2, wherein it further comprises a cable arm supporting cables for X-ray tube and image receiver mounted on the C-shaped bracket, and which is arranged to be always directed upwards and to keep the cables above the main arm when it is rotated.

5. An X-ray apparatus according to any of the preceding claims, wherein the C-shaped bracket in a position at one end of the table is arranged to be rotated in its own plane to a position, where the ray path between X-ray tube and image receiver has a substantially horizontal orientation, and thereafter to be rotated 180° about its suspension on the base unit, and then again to be rotated back to its own plane to a position where the ray path has a substantially vertical orientation.

6. An X-ray apparatus according to any of the preceding claims, wherein the table may rotate freely about its suspension on the lateral arm.

7. An X-ray apparatus according to any of the preceding claims, wherein the apparatus comprises a control unit for controlling the desired movements and which is provided with pre-programmed stop codes for the movement of the different parts of the apparatus, the movements depending on the physical dimensions of the parts of the apparatus and on the room in which the apparatus is situated.

8. An X-ray apparatus according to any of the claims 4 - 7, wherein the cable arm is mounted on the C-shaped bracket at a point close to the suspension of the bracket on the base unit.

9. A method for rearranging an X-ray apparatus comprising a base unit from which an L-shaped angular bracket is suspended, the angular bracket comprising a main arm carrying through a suspension a C-shaped bracket with an X-ray tube at one end and an image receiver at the other end, where a lateral arm mounted on the C-shaped bracket carries a patient table for supporting a person, and where the C-shaped bracket and the angular bracket are arranged for mutual movement in the form of rotation and/or translation, **characterised** in that the table is put in a starting position where the ray path extends along a substantially straight line through the table and where the table is mounted on the lateral arm in a first horizontal position in parallel with the main arm and with the head end directed in a first direction, that the table is displaced to a position outside the C-shaped bracket, that the table is rotated to a second horizontal position in parallel with the main arm, in which position the head end of the table is directed opposite to the first direction, and that the table is displaced into the ray path.

10. A method according to claim 9 for rearranging an X-ray apparatus of the kind where the table is mounted on a lateral arm connected to the main arm with a suspension at one end, wherein the displacement of the table from the first to the second horizontal position takes place by rotating the main arm and the table mounted thereon through an angle of 180° about an axis which is parallel with the axis of rotation of the C-shaped bracket, that the table is rotated 180° about its longitudinal axis, and that the table is displaced into the ray path.

11. A method according to claim 9 for rearranging an X-ray apparatus of the kind where the table is mounted on a lateral arm, which is sliding along the main arm, either with the C-shaped bracket or independently of the C-shaped bracket, wherein the table is slided in a direction in parallel with the main arm until it is outside the ray path, that the table is rotated 180° about a vertical axis, and that the table subsequently is slided into the ray path.

12. A method according to any of the claims 9 -11, wherein the C-shaped bracket, when the table has been displaced outside the ray path, is rotated 90° in its own plane so that the ray path extends substantially horizontally, that the C-shaped bracket is rotated 90° in its own plane in the opposite direction, and that the table is slided into the ray path where the positions of the X-ray tube and the image receiver are now interchanged.

13. A method according to claim 9 or 10, wherein the table is rotated to a vertical position after displacing it to the position outside the C-shaped bracket as the table is rotated about a longitudinal axis in parallel with the main arm.
